## Europäisches Patentamt
## European Patent Office
## Office européen des brevets

(19)

(11) Publication number: **0 006 746**
**B1**

(12) **EUROPEAN PATENT SPECIFICATION**

(45) Date of publication of patent specification: **02.06.82**

(51) Int. Cl.³: **G 01 N 33/18**

(21) Application number: **79301214.7**

(22) Date of filing: **22.06.79**

(54) **An apparatus for and a process of detecting pollution in an aqueous medium.**

(30) Priority: **22.06.78 GB 2760178**

(43) Date of publication of application:
**09.01.80 Bulletin 80/1**

(45) Publication of the grant of the patent:
**02.06.82 Bulletin 82/22**

(84) Designated Contracting States:
**DE FR GB IT NL**

(56) References cited:
**DE - A - 2 104 969**
**DE - A - 2 132 166**
**FR - A - 2 400 200**
**US - A - 3 807 860**
**US - A - 4 057 721**

**BBC — NACHRICHTEN, Vol. 58, Nr. 2/3, 1976, Mannheim D. SCHNABEL, "Physikalische Messverfahren zur Bestimmung der Wasserqualität" pages 73 to 84.**

(73) Proprietor: **Esso Société Anonyme Française**
**6 Avenue André Prothin**
**F-92400 Courbevoie (FR)**

(72) Inventor: **Akoun, Gerald Lazare**
**16 Rue de Roumois**
**F-76130 Mont Saint Aignan (FR)**
Inventor: **Muller, Jean-Marie**
**26 Rue Maurice Utrillo**
**F-76420 Bihorel (FR)**

(74) Representative: **Field, Roger Norton**
**Hanover Court 5 Hanover Square**
**London W1R OHQ (GB)**

## An apparatus for and a process of detecting pollution in an aqueous medium

This invention relates to apparatus for detecting pollution of an aqueous effluent by soluble organic or inorganic compounds.

German Patent Specification 2104969 discloses a fluid testing apparatus wherein the conductivity, pH value etc of water can be detected. German Patent Specification 2132166 discloses apparatus for testing water, sewage, etc. French Patent Specification 2400200 discloses a process and apparatus for analysing aqueous effluent and river waters. Contrary to the apparatus of our invention, none of this prior art discloses the use of a U.V. detector and hence fails to disclose the method of detecting and analysing the amount of hydrocarbons in the aqueous effluent. Neither do they disclose the use of a device such as a charcoal filter for absorbing hydrocarbons.

Furthermore, there is a need for an apparatus or a device which enables one continuously to take aqueous samples of sewage and to determine the extent of accidental pollution by pollutants. We have now devised an apparatus for this purpose and which can be adapted to set off quickly an alarm so that adequate remedial action can be taken.

According to this invention, an apparatus for detecting pollution of an aqueous effluent comprises:

(a) a sampling device,

(b) a reservoir for aqueous effluent,

(c) a filter upstream of said reservoir (b),

(d) a filter downstream of said reservoir (b),

(e) a reservoir for water of known purity,

(f) a filter downstream of said reservoir (e),

(g) a thermostatically controlled bath connected by conduit to both reservoirs (b) and (e),

(h) a coalescer downstream of said bath (g),

(i) a device for absorbing hydrocarbons connected by conduit to coalescer (h),

(j) either a differential refractive index detector connected by conduit to coalescer (h), or a conductimeter connected by conduit to bath (g) or by conduit to coalescer (h),

(k) a U.V. detector downstream of said device (i),

(l) means for conveying water of known purity from its reservoir (e) through filter (f), bath (g), coalescer (h), device (i), detector (k) and detector or conductimeter (j), and

(m) means for conveying a sample of aqueous effluent from its reservoir (b) through filter (d), bath (g), coalescer (h), device (i), detector (k) and detector or conductimeter (j).

By comparing the readings obtained in the detector or conductimeter (j) and detector (k), with on the one hand water of known purity and on the other hand effluent one is able to determine at least qualitatively the pollution in the effluent.

Before the water of known purity (hereinafter referred to as "clean water") and effluent are tested they must be collected in reservoirs. There are separate reservoirs for clean water and for effluent. By means of valves and a pump, the clean water and effluent can be separately conveyed to their respective reservoirs. The effluent and optionally clean water should also pass through filters before they pass to their reservoirs and also after they leave their reservoirs. A sample of the aqueous effluent is transferred to its reservoir (b) by means of the sampling device (a), and this conveniently is achieved by means of a suction/compression device to be described in more detail later.

The clean water reservoir (e) may be provided with a level detector which operates the valve allowing clean water to enter its reservoir (e). Also the conduit through which the clean water passes on its way to the reservoir (e) may be provided with a security device which stops the apparatus should clean water not be available.

The clean water which may be for example tap water, deionized water or distilled water supplies both its reservoir and the thermostatically controlled bath. After leaving its reservoir and after passing its filter the clean water enters the thermostatically controlled bath and (g) the conductimeter (j) (when used) and then the coalescer (h). This bath controls the temperature of the water going through the coalescer (h) and device (i). This bath may also be used, if needed for the thermostatic control of the conductimeter (j) and of detector (k). Any overflow of clean water from the thermostatically controlled bath (g) can be returned back to the clean water reservoir.

The means (l) for conveying clean water is usually a pump and by this means clean water is pumped from its reservoir through bath (g) to the conductimeter (j) (when used) and coalescer (h). A first part of the water leaving the coalescer (h) is removed from the system, being sent to a sewer. A second part of the water leaving the coalescer (h) is split between entering the device (i) through the conduit connecting the device (i) to the coalescer (h) and entering either the differential refractive index detector (j) (when used) through the conduit connecting it to the coalescer (j) or the U.V. detector (k) (when the differential refractive index detector is not used) through a conduit connecting it to the coalescer (h). Water emerging from the device (i) is conveyed either to the differential refractive index detector (j) (when used) or to the U.V. detector (k) (when the differential refractive index detector is not used).

The coalescer (h) which coalesces droplets is usually a tube filled with resin. It can however be a tube filled with glass or organic fibres, polyurethane foam or other coalescing material. The device (i) for absorbing hydrocarbons is usually a charcoal filter, and will hereinafter be referred to as the charcoal filter.

The stream leaving the charcoal filter (i) is sent to the reference cells of the detector (j) (when used) and to the U.V. detector (k). It also enables one to detect on U.V. detector (k) saturation of the charcoal filter (i) because when products are not absorbed on the charcoal filter a negative variation will appear on the recorder· of the U.V. detector (k).

The aqueous effluent whose pollution is to be detected is sampled for example from a sewer by the sampling device which is usually a suction/compression device. By this means it is possible to suck up the effluent into its reservoir (b) until a certain level is achieved. The sample is then allowed to decant in the reservoir (b).

At various intervals, part of the aqueous effluent is passed from its reservoir (b). In a preferred embodiment it is passed through a pH-meter which acts as a first control of the aqueous effluent. If the pH-value detected is outside a predetermined pH range, the value is recorded and the reservoir (b) is emptied under pressure by the suction/compression device and the pH-meter is cleaned by clean water. Then, the reservoir (b) is charged with new aqueous effluent sample.

When the measured pH of the aqueous effluent is within the predetermined pH range, a measured part of the aqueous effluent is passed from its reservoir (b) through the above-described system instead of clean water. These intervals may for example be at 5 to 30 minutes. The amount of the sample and these intervals can be adjusted depending on the pollution level of the aqueous effluent. When the sample of the aqueous effluent has been removed from its reservoir (b), means are provided for automatically supplying clean water to the apparatus. Also during the analysis of the aqueous effluent, the aqueous effluent reservoir (b) is emptied under pressure by the suction/compression device and charged with new aqueous effluent sample. If a pH meter is used the meter is cleaned.

When a conductimeter (j) is used, the measured part of the aqueous effluent passes through the thermostatically controlled bath (g) for temperature adjustment and then into the conductimeter (j) where the conductivity is measured and recorded giving information as to how much salt there is.

The remainder of the aqueous effluent emerging from the coalescer (h) contains soluble products and is split into two streams (as with the clean water), a first stream entering the charcoal filter (i) and a second stream entering the U.V. detector (k) or when used, the differential refractive index detector (j).

In the first alternative, i.e. when the second stream enters the U.V. detector, in said U.V. detector (k), the second stream is compared with the first stream after it passed through the charcoal filter (i). Since the charcoal filter (i) absorbs soluble hydrocarbons, this comparison is made with clean water. The U.V. detector (k) gives an analysis of the effluent, e.g. at 254 nm, it gives an indication of the presence of hydrocarbons (other than paraffins) and all oxygenated products.

Similarly in the second alternative when the second stream enters the differential refractive index detector (j), in said detector (j) the second stream is compared with the first stream after it has passed through the charcoal filter (i). Since the charcoal filter absorbs soluble hydrocarbons this comparison is made with clean water. The differential refractive index detector (j) gives a general idea of the pollution of the effluent whereas the U.V. detector can give an analysis of the effluent, e.g. at 254 nm it gives an indication of the presence of hydrocarbons (other than paraffins) and all oxygenated products.

Since inorganic soluble products are not absorbed on the charcoal, they pass through and are detected by the differential refractive index detector (j). A negative variation will appear on the recorder. Due to the difference in the passage time the two variations on the recorder are well indicated.

As an alternative to positioning the conductimeter (j) immediately downstream of the bath (g) it may be positioned downstream of the coalescer (h) provided it is upstream of the split of the exit line from the coalescer (h) i.e. before the line divides into entering the charcoal filter (i) and the U.V. detector (k).

All the information obtained from the pH-meter, the conductimeter and U.V. detector can be recorded and the apparatus can be monitored by installing limits and alarms e.g. lamps, bells etc., which inform people about the variation of the composition of the stream or maintenance problems.

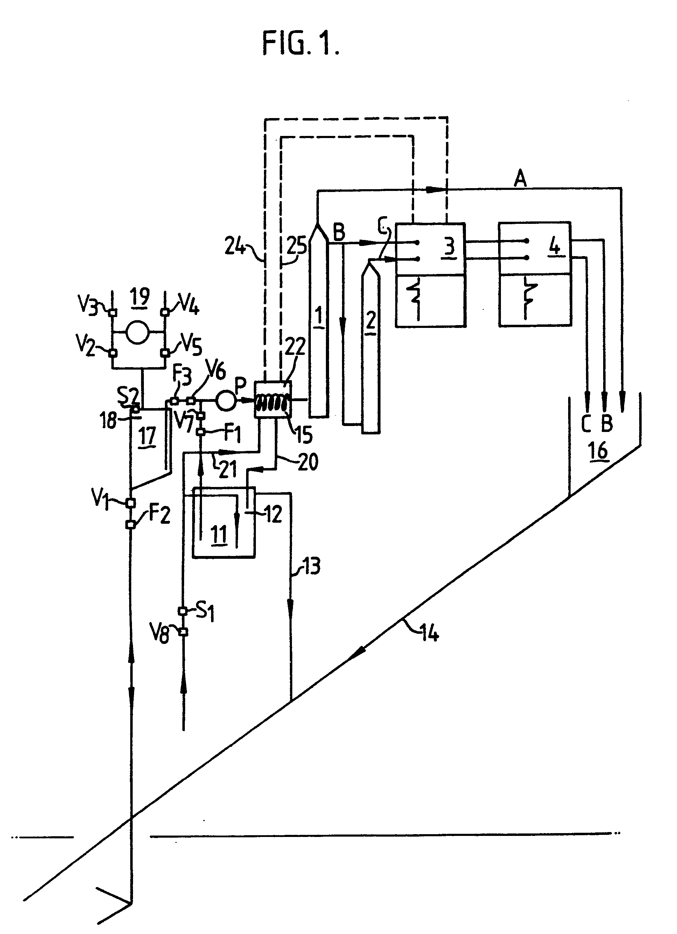

A particular form of apparatus according to the invention is now described with reference to Fig. 1 of the accompanying drawing.

Clean water enters its reservoir 11 via an electro valve $V_8$ and a security device $S_1$. This reservoir is provided with a level detector 12. Overflow water passes through conduit 13 to the sewer pipe 14. Clean water is sucked up through filter $F_1$, electro valve $V_7$ by means of pump P and enters a coil 22 in the thermostatically controlled bath 15. From this coil 22 clean water is conveyed to coalescer 1. Water leaves the coalescer 1 by line B, and enters both the charcoal filter 2 and the differential refractive index detector 3 and then the U.V. detector 4. Water emerging from charcoal filter 2 also passes to detector by line C. Another stream A leaves the coalescer 1 and is sent to the sewer 16. Clean water also enters bath 15 by means of line 21 and returns to the reservoir 11 by means of line 20. Heat exchange occurs between this water and that passing through coil 22. If the temperature of the detectors has to be controlled, the thermostatically controlled bath 15 supplies warm water for example, through dotted line 24 and returns through dotted line 25.

Aqueous effluent is drawn up to its reservoir 17 through a filter $F_2$ and electro valve $V_1$. The reservoir 17 has a level detector 18 and a security device $S_2$ and is connected to a suction/compression device 19 having electro valves $V_2$, $V_3$, $V_4$ and $V_5$.

After leaving the reservoir 17 via filter $F_3$, electro valve $V_6$ and pump P the aqueous effluent passes through the coil 22 in the thermostatically controlled bath 15 and onto the coalescer 1, charcoal filter 2 and detectors 3 and 4 and to sewer 16 in the same way as described in connection with the clean water.

Clean water is continuously drawn up to its reservoir 11 and to the thermostatically controlled bath 15. When the required level in reservoir 11 is reached, the level detector shuts off the electro valve $V_8$. The security device $S_1$ stops the apparatus should there be no clean water available to be drawn into the reservoir 11. Clean water is then drawn up by means of pump P through filter $F_1$, and electro valve $V_7$ and through the coil 22 in the bath 15 to the coalescer 1, charcoal filter 2, detectors 3 and 4 and sewer 16 as previously described. Overflow from bath 15 returns to reservoir 11 by means of line 20.

From time to time, aqueous effluent is sent through the apparatus instead of clean water. This is achieved as follows: The suction/compression device 19 comprises a small air compressor with four electrovalves $V_2$, $V_3$, $V_4$ and $V_5$. When $V_2$ and $V_4$ are open and $V_3$ and $V_5$ closed, suction is applied to the reservoir 17. When $V_2$ and $V_4$ are closed and $V_3$ and $V_5$ are open, compression is applied to the reservoir 17. The reservoir 17 is provided with a security device $S_2$ which prevents high vacuum or high pressure. When valves $V_2$ and $V_4$ are open and suction occurs in the reservoir 17 and when the water level reaches the level of the level detector 18 the compressor stops and the electro valves $V_1$, $V_2$ and $V_4$ are closed. There is then a decantation phase in the reservoir 17. When programmed a sample of aqueous effluent is removed from the reservoir 17, electro valve $V_7$ having been switched off and electro valve $V_6$ switched on. When this sample has been removed from the reservoir 17, the electro valve $V_6$ is closed and electro valves $V_1$, $V_3$, $V_5$ and $V_7$ are opened. This enables the reservoir 17 to be emptied and for the filter $F_2$ to be cleaned and clean water sent through the apparatus once more.

The sample of aqueous effluent obtained from the reservoir 17 then enters the thermostatically controlled bath 15 and then to the coalescer 1 where insoluble hydrocarbons are separated. These insoluble products are sent back to the sewer 16 by stream A. Stream B emerging from the coalescer 1 contains soluble products and is split between the detectors 3 and 4 and the charcoal filter 2.

In the differential refractive index detector 3, the stream B is compared with the clean water C emerging from the charcoal filter 2. From this comparison one is able to obtain a qualitative idea of the total pollution of the effluent and of the inorganic soluble products. The U.V. detector 4 enables one to determine the presence of hydrocarbons other than paraffins and all oxygenated compounds.

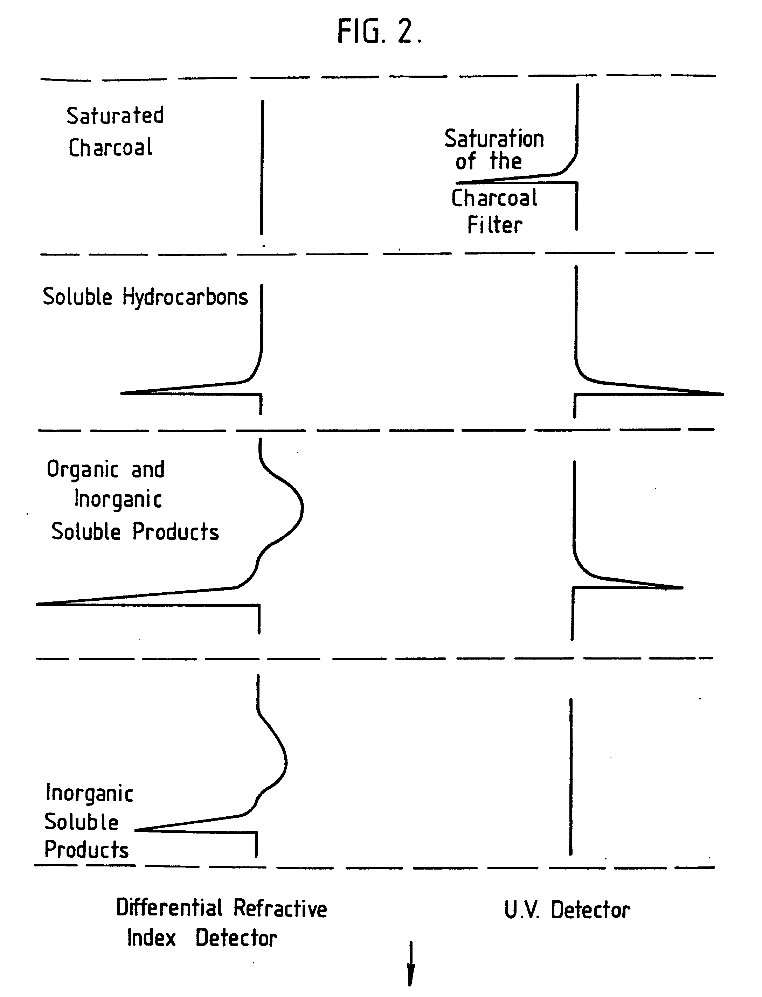

Fig. 2 shows typical readings obtained on the chart recorder from the differential refractive index detector (3) and from the U.V. detector (4).

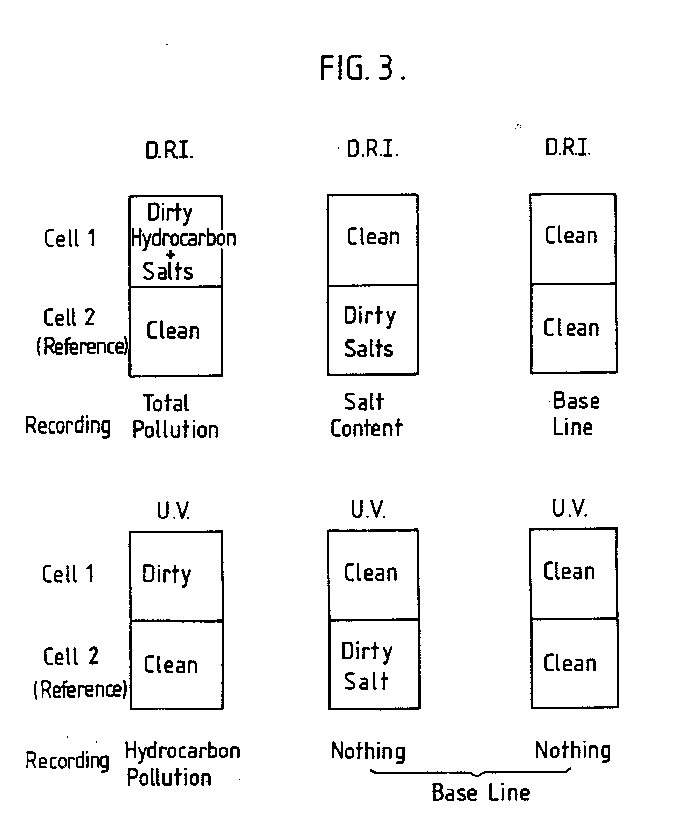

Fig. 3 shows the typical sequence of liquid passing through the differential refractive index detector 3 and the U.V. detector 4. Thus, it can be seen how these detectors can detect the presence of soluble hydrocarbons and organic and inorganic soluble products. It is worth reiterating that in the coalescer all insoluble organic products are removed and can be sent to the sewer, e.g. via line A. Effluent containing salts plus soluble hydrocarbons is sent to detectors 3 and 4 where there are comparisons with clean water. In the next step in the cycle the nature of the liquids entering the detectors 3 and 4 are reversed and the presence of soluble salt in the effluent can be detected. In the final step of this cycle, clean water enters the detectors 3 and 4 and this forms the base line for the detectors.

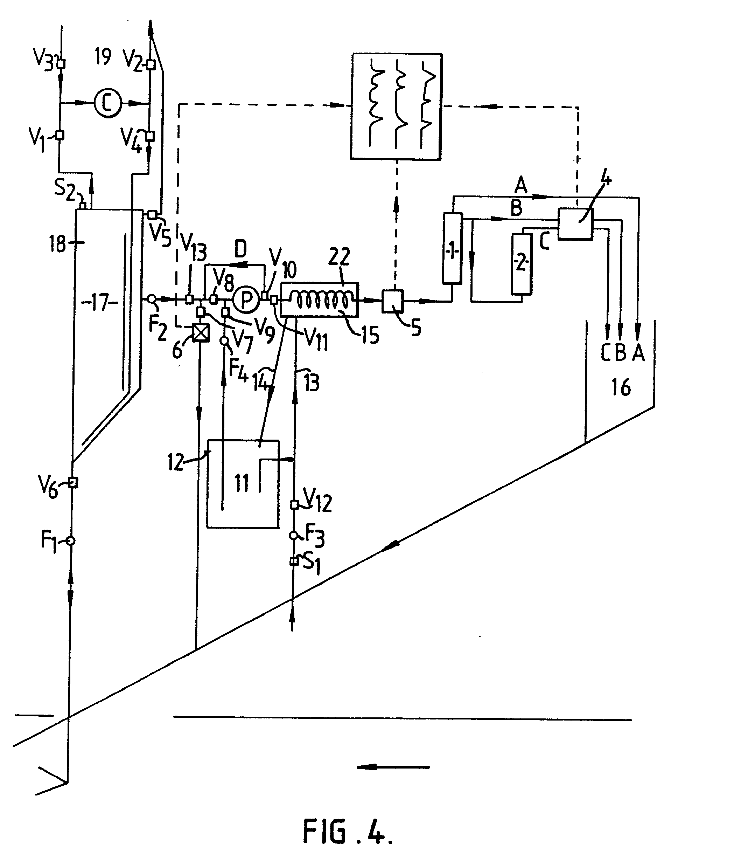

Another form of apparatus according to the invention is now described with reference to Fig. 4 of the accompanying drawings.

Clean water enters its reservoir 11 via a security device $S_1$, a filter $F_3$ and an electrovalve $V_{12}$. This reservoir is provided with a level detector 12. Electrovalves $V_8$ and $V_{10}$ having been switched off, clean water is sucked through filter $F_4$, electrovalve $V_9$ by means of pump P and enters a coil 22 in the thermostatically-controlled bath 15. From this coil 22, clean water is conveyed through a conductimeter 5 and then to a coalescer 1. Water leaves the coalescer 1 by line B. The line is split into two parts, the first going to the U.V. detector 4 and the second (line C) going through the charcoal filter 2 and the U.V. detector 4. Water emerging from the charcoal filter 2 also passes to detector 4 by line C. Another stream A leaves the coalescer 1 and is sent to the sewer 16. Clean water also enters bath 15 by means of line 13 and overflow returns to reservoir 11 by means of line 14. Heat exchange occurs between the water in the bath and that passing through coil 22.

Aqueous effluent is drawn up to its reservoir 17 through a filter $F_1$ and electrovalve $V_6$. The reservoir 17 has a level detector 18 and a security device $S_2$ and is connected to a suction/compression device 19 having electrovalves $V_1$, $V_2$, $V_3$ and $V_4$ and an air compressor C. The sampled aqueous effluent water can be stripped in the reservoir 17 and the gas sent out through the electrovalve $V_5$.

After leaving by gravity the reservoir 17 via filter $F_2$, the aqueous effluent passes through an electrovalve $V_7$ and a pH-meter 6 and is sent to the sewer 16. When the measured pH is inside the predetermined pH range, the aqueous effluent, after leaving the reservoir 17 via filter $F_2$, electrovalve $V_8$, pump P and electrovalve $V_{11}$, passes through the coil 22 in the thermostatically-controlled bath 15 and onto the conductimeter 5, coalescer 1, charcoal filter 2 and detector 4, and to sewer 16, in the same way as described in connection with the clean water. Line D and electrovalve $V_{10}$ are used for cleaning the pH-meter 6.

Clean water is continuously drawn up to its reservoir 11 and to the thermostatically-controlled bath 15. When the required level in reservoir 11 is reached, the level detector shuts off the electrovalve $V_{12}$. The security device $S_1$ stops the apparatus should there be no clean water available to be drawn into the reservoir 11. Clean water is then drawn up by means of pump P through filter $F_4$ and electrovalve $V_9$ and through the coil 22 in the bath 15 to the conductimeter 5, the coalescer 1, the charcoal filter 2, the detector 4 and the sewer 16 as previously described. Overflow from bath 15 returns to reservoir 11 by means of line 14.

From time to time, aqueous effluent is sent through the apparatus instead of clean water. This is achieved as follows: the suction/compression device 19 comprises a small air compressor with four electrovalves $V_1$, $V_2$, $V_3$, $V_4$. When $V_1$ and $V_2$ are open and $V_3$, $V_5$ and $V_{13}$ are closed, suction is applied to the reservoir 17. When $V_1$, $V_2$, $V_5$, $V_{13}$ are closed and $V_3$, $V_4$ are open, compression is applied to reservoir 17. The reservoir 17 is provided with a security device which prevents high vacuum or high pressure. When valves $V_1$, $V_2$ and $V_6$ are open and $V_3$, $V_4$, $V_5$ and $V_{13}$ are closed, suction occurs in the reservoir 17, effluent is drawn into the reservoir and when the water level reaches the level of the level detector 18 the compressor stops and the electrovalves $V_6$, $V_1$ and $V_2$ are closed. There is then a stripping phase in the reservoir 17, the electrovalves $V_3$, $V_4$ and $V_5$ are open and the compressor running. When programmed, a sample of aqueous effluent is removed by gravity from the reservoir 17, electrovalves $V_7$ and $V_{13}$ having been switched on, the aqueous effluent passes through the pH-meter 6 and is sent to the sewer 16.

As previously described, depending on the pH value, the electrovalves $V_7$ and $V_9$ are switched off and electrovalve $V_8$ is switched on and then a sample of aqueous effluent is removed by means of the pump from the reservoir for conductivity and U.V. determinations.

When the sample has been removed from the reservoir 17, the electrovalves $V_1$, $V_8$ and $V_{13}$ are switched off and electrovalves $V_1$, $V_2$, $V_6$ and $V_9$ are switched on and the compressor run. This enables the reservoir 17 to be emptied under pressure and for the filter $F_1$ to be cleaned and clean water sent through the apparatus once more.

Then the compressor is stopped and electrovalves $V_{11}$ and $V_{13}$ are switched off and electrovalves $V_7$ and $V_{10}$ are switched on, this allows the cleaning of the pH-meter 6. After cleaning the pH-meter, the electrovalve $V_{10}$ is switched off and the electrovalve $V_{11}$ is switched on and clean water sent through the apparatus.

The sample of aqueous effluent obtained from the reservoir 17 then enters the thermostatically-controlled bath 15 and then the conductimeter where the conductivity is measured and then the coalescer 1 where the insoluble hydrocarbons are separated. These insoluble products are sent back to the sewer 16 by stream A. Stream B emerging from the coalescer 1 contains soluble products and is split between the U.V. detector 4 and the charcoal filter 2.

The U.V. detector 4 enables one to determine the presence of soluble hydrocarbons.

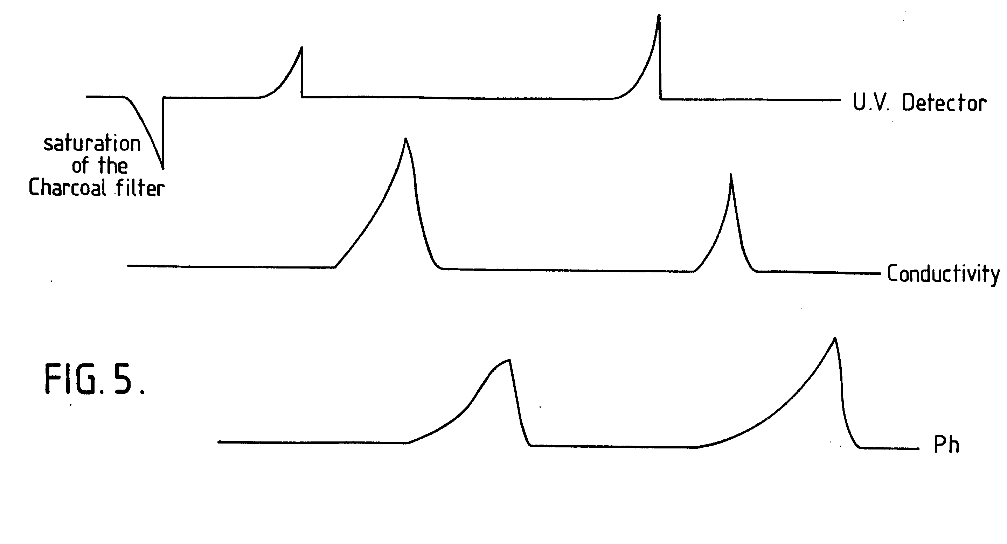

Fig. 5 shows typical reading obtained on the chart from the pH-meter, conductimeter, and from the U.V. detector.

The apparatus of this invention can be used in for example refineries, chemical plants, storage plants, and any industrial plants where it is desired to detect pollution in aqueous effluent.

**Claims**

1. An apparatus for detecting pollution of an aqueous effluent which comprises:
(a) a sampling device,
(b) a reservoir for aqueous effluent,
(c) a filter upstream of said reservoir (b),
(d) a filter downstream of said reservoir (b),
(e) a reservoir for water of known purity,
(f) a filter downstream of said reservoir (e),
(g) a thermostatically controlled bath connected by conduit to both reservoirs (b) and (e),
(h) a coalescer downstream of said bath (g),
(i) a device for absorbing hydrocarbons connected by conduit to (h),
(j) either a differential refractive index detector connected by conduit to coalescer (h), or a conductimeter connected by conduit to bath (g) or by conduit to coalescer (h),
(k) a U.V. detector downstream of device (i),
(l) means for conveying water of known purity from its reservoir (e) through filter (f), bath (g), coalescer (h), device (i), detector (k) and detector or conductimeter (j), and
(m) means for conveying a sample of a aqueous effluent from its reservoir (b) through filter (d), bath (g), coalescer (h), device (i), detector (k) and detector or conductimeter (j).

2. An apparatus according to claim 1, wherein the sampling device (a) comprises a suction/compression device.

3. An apparatus according to either of claims 1 and 2), wherein the water reservoir (e) is provided with a level detector.

4. An apparatus according to any one of the preceding claims, wherein the means (I) for conveying water of known purity is a pump.

5. An apparatus according to any one of the preceding claims which includes means for automatically supplying water of known purity to the apparatus when aqueous effluent has been removed from its reservoir.

6. An apparatus according to any one of the preceding claims, wherein a conductimeter is used and there is a pH meter connected to the reservoir (b).

7. An apparatus according to any one of the preceding claims which includes a filter located upstream of reservoir (e).

8. A process of detecting pollution in an aqueous effluent which comprises passing intermittently said effluent through the apparatus according to any one of claims 1 to 5 in which a differential refractive index detector is used and at other times conveying continuously water of known purity through said apparatus and comparing readings obtained from the differential refractive index detector (j) and from the U.V. detector (k).

9. A process according to claim 8, wherein a first part of water leaving coalescer (h) is removed from the system and the second part of water leaving coalescer (h) is split between entering the device (i) and entering the differential refractive index detector (j).

10. A process of detecting pollution of an aqueous effluent which comprises passing intermittently said effluent through the apparatus according to claim 6 and at other times conveying continuously water of known purity through said apparatus and comparing readings obtained from the pH-meter, the conductimeter (j) and the U.V. detector (h).

11. A process according to claim 10, wherein a first part of the water leaving the coalescer (h) is removed from the system and the second part of water leaving the coalescer (h) is split between entering the device (i) and entering the U.V. detector (h).

**Revendications**

1. Appareil pour la détection de pollution d'un effluent aqueux qui comprend:
(a) un dispositif d'échantillonnage,
(b) un réservoir pour l'effluent aqueux,
(c) un filtre en amont du réservoir (b),
(d) un filtre en aval du réservoir (b),
(e) un réservoir pour de l'eau de pureté connue,
(f) un filtre en aval du réservoir (e),
(g) un bain commandé thermostatiquement relié par un conduit aux deux réservoirs (b) et (e),
(h) un dispositif de combinaison placé en aval du bain (g),
(i) un dispositif d'absorption d'hydrocarbures relié par un conduit au dispositif de combinaison (h),
(j) un détecteur d'indice de réfraction différentiel relié par un conduit au dispositif de combinaison (h), ou un dispositif de mesure de conductivité relié par un conduit au bain (g) ou par un conduit au dispositif de combinaison (h),
(k) un détecteur d'ultraviolets placé en aval du dispositif (i),
(l) un moyen pour faire passer l'eau de pureté connue de son réservoir (e) par le filtre (f), le bain (g), le dispositif de combinaison (h), le dispositif (i), le détecteur (k) et le détecteur ou le dispositif de mesure de conductivité (j), et
(m) un moyen pour faire passer un échantillon d'effluent aqueux de son réservoir (b) par le filtre (d), le bain (g), le dispositif de combinaison (h), le dispositif (i), le détecteur (k) et le détecteur ou le dispositif de mesure de conductivité (j).

2. Appareil selon la revendication 1, dans lequel le dispositif d'échantillonnage (a) est constitué par un dispositif d'aspiration/compression.

3. Appareil selon l'une quelconque des revendications 1 et 2 dans lequel le réservoir d'eau (e) est pourvu d'un détecteur de niveau.

4. Appareil selon l'une quelconque des revendications 1 à 3 dans lequel le moyen (l) pour refouler de l'eau de pureté connue est une pompe.

5. Appareil selon l'une quelconque des revendications 1 à 4 qui comprend un moyen pour fornir automatiquement de l'eau de pureté connue à l'appareil quand l'effluent aqueux a été retiré de son réservoir.

6. Appareil selon l'une quelconque des revendications 1 à 5 dans lequel un dispositif de mesure de conductivité est utilisé et dans lequel un dispositif de mesure de pH est relié au réservoir (b).

7. Appareil selon l'une quelconque des revendications 1 à 6 qui comprend un filtre situé en amont du réservoir (e).

8. Procédé pour la détection de pollution dans un effluent aqueux, qui consiste à faire passer par intermittence l'effluent aqueux dans l'appareil selon l'une quelconque des revendications 1 à 5 dans lequel est utilisé un détecteur d'indice de réfraction différentiel et à d'autres moments à faire passer de façon permanente de l'eau de pureté connue dans l'appareil et à comparer les valeurs obtenues à partir du détecteur d'indice de réfraction différentiel (j) et à partir du détecteur d'ultraviolets (k).

9. Procédé selon la revendication 8 dans lequel une première partie de l'eau sortant du dispositif de combinaison (h) est retirée du système et la seconde partie de l'eau quittant le dispositif de combinaison (h) est répartie entre l'entrée du dispositif (i) et l'entrée du détecteur d'indice de réfraction différentiel (j).

10. Procédé de détection de pollution d'un effluent aqueux, qui consiste à faire passer de façon intermittente l'effluent dans l'appareil selon la revendication 6 et à d'autres moments à faire passer de façon permanente de l'eau de pureté connue dans l'appareil et à comparer les valeurs obtenues à partir du dispositif de mesure de pH, du dispositif de mesure de conductivité (j) et du détecteur d'ultraviolets (k).

11. Procédé selon la revendication 10 dans lequel une première partie de l'eau sortant du dispositif de combinaison (h) est retirée du système et la seconde partie de l'eau quittant le dispositif de combinaison (h) est répartie entre l'entrée du dispositif (i) et l'entrée du détecteur d'ultraviolets (k).

## Patentansprüche

1. Vorrichtung zur Bestimmung der Verschmutzung von Abwasser gekennzeichnet durch:
a) einen Probennehmer,
b) ein Vorratsgefäßt für Abwasser,
c) einen Filter, der sich stromaufwärts vom Vorratsgefäß (b) befindet,
d) einen Filter, der sich stromabwärts vom Vorratsgefäß (b) befindet,
e) ein Vorratsgefäß für Wasser bekannter Reinheit,
f) einen Filter, der sich stromabwärts vom Vorratsgefäß (e) befindet,
g) ein mittels eines Thermostaten reguliertes Bad, das über Leitungen mit den beiden Vorratsgefäßen (b) und (e) verbunden ist,
h) einen Koalisierer, der sich stromabwärts vom Bad (g) befindet,
i) eine Vorrichtung zur Absorption von Kohlenwasserstoffen, die über eine Leitung mit (h) verbunden ist,

j) entweder einen Differentialrefraktionsindexdetektor, der über eine Leitung mit dem Koalisierer (h) verbunden ist, oder ein Konduktometer, das über eine Leitung mit dem Bad (g) oder dem Koalisierer (h) verbunden ist,
k) einen UV-Detektor, der sich stromaufwärts von Vorrichtung (i) befindet,
l) Mittel zur Beförderung von Wasser bekannter Reinheit aus seinem Vorratsgefäß (e) durch Filter (f), Bad (g), Koalisierer (h), Vorrichtung (i), Detektor (k) und Detektor oder Konduktometer (j), und
m) Mittel zur Beförderung ein Abwasserprobe aus dem Abwasservorratsgefäß (b) durch Filter (d), Bad (g), Koalisierer (h), Vorrichtung (i), Detektor refraktionsindexdetektor (j) und vom UV-Detektor (k) erhaltenen Ablesungen verglichen werden.

9. Verfahren nach Anspruch 8, dadurch gekennzeichnet, daß ein erster Teil des den Koalisierer (h) verlassenden Wassers aus dem System entfernt wird und der zweite Teil des den Koalisierer (h) verlassenden Wassers zwischen Eintritt in die Vorrichtung (i) und Eintritt in den Differentialrefraktionsindexdetektor (j) aufgeteilt wird.

10. Verfahren zur Bestimmung der Verschmutzung von Abwasser, dadurch gekennzeichnet, daß das Abwasser intermittierend durch die Vorrichtung gemäß Anspruch 6 geleitet wird, zu den anderen Zeiten kontinuierlich Wasser bekannter Reinheit durch die Vorrichtung geleitet wird und die vom pH-Meter, vom Konduktometer (j) und vom UV-Detektor (h) erhaltenen Ablesungen verglichen werden.

11. Verfahren nach Anspruch 10, dadurch gekennzeichnet, daß ein erster Teil des den Koalisierer (h) verlassenden Wassers aus dem System entfernt wird und der zweite Teil des den Koalisierer (h) verlassenden Wassers zwischen Eintritt in die Vorrichtung (i) und Eintritt in den UV-Detektor (h) aufgeteilt wird.

# FIG. 1.

**0 006 746**

# FIG. 2.

Saturated Charcoal

Saturation of the Charcoal Filter

Soluble Hydrocarbons

Organic and Inorganic Soluble Products

Inorganic Soluble Products

Differential Refractive Index Detector

U.V. Detector

2

# FIG. 3.

D.R.I.

| | |
|---|---|
| Cell 1 | Dirty Hydrocarbon + Salts |
| Cell 2 (Reference) | Clean |

Recording — Total Pollution

D.R.I.

| | |
|---|---|
| | Clean |
| | Dirty Salts |

Salt Content

D.R.I.

| | |
|---|---|
| | Clean |
| | Clean |

Base Line

U.V.

| | |
|---|---|
| Cell 1 | Dirty |
| Cell 2 (Reference) | Clean |

Recording — Hydrocarbon Pollution

U.V.

| | |
|---|---|
| | Clean |
| | Dirty Salt |

Nothing

U.V.

| | |
|---|---|
| | Clean |
| | Clean |

Nothing

Base Line

FIG .4.

FIG. 5.

U.V. Detector

saturation
of the
Charcoal filter

Conductivity

Ph

0 006 746